**Europäisches Patentamt**

**European Patent Office**

**Office européen des brevets**

(19)

(11)  **EP 1 059 355 A1**

(12)  **DEMANDE DE BREVET EUROPEEN**

(43) Date de publication:
**13.12.2000 Bulletin 2000/50**

(51) Int Cl.⁷: **C12N 15/31**, C07K 14/24,
A01N 63/02

(21) Numéro de dépôt: **99870124.7**

(22) Date de dépôt: **11.06.1999**

(84) Etats contractants désignés:
**AT BE CH CY DE DK ES FI FR GB GR IE IT LI LU MC NL PT SE**
Etats d'extension désignés:
**AL LT LV MK RO SI**

(71) Demandeur: **Agrostar**
**1300 Wavre (BE)**

(72) Inventeurs:
• **Thonart, Philippe**
**B-5081 La Bruyère (BE)**
• **Jabrane, Abdelhamid**
**B-4020 Liège (BE)**
• **Destain, Jacqueline**
**B-1450 Cortil Noirmont (BE)**

• **Pierrard, Annick**
**B-4690 Bassenge (BE)**
• **Drion, Raphael**
**B-5520 Onhaye (BE)**
• **Jacques, Philippe**
**B-4000 Liège (BE)**

(74) Mandataire: **Claeys, Pierre et al**
**Gevers & Vander Haeghen,**
**Patent Attorneys,**
**Rue de Livourne 7**
**1060 Brussels (BE)**

Remarques:
The applicant has subsequently filed a sequence listing and declared, that it includes no new matter.

(54)  **Bactériocine, sa préparation et son utilisation**

(57)  Bactériocine appartenant au groupe des bactériocines de haut poids moléculaire (BHPM), caractérisée en ce qu'elle comprend au moins une première sous-unité majeure dont l'extrémité amino-terminale présente une homologie avec une séquence amino-terminale de protéine de tube de phage 186 (SEQ ID N°2), et une seconde sous-unité majeure dont l'extrémité amino-terminale présente une homologie avec une séquence amino-terminale de protéine de manchon de phage P2 (SEQ ID N°4).

FIG. 1

EP 1 059 355 A1

**Description**

La présente invention se rapporte à une bactériocine appartenant au groupe des bactériocines de haut poids moléculaire (BHPM) , à la souche de Serratia Plymuthica dont elle provient et à l'utilisation de ladite bactériocine.

Les bactériocines sont des molécules bactéricides produites par certaines souches bactériennes, dont le spectre d'action se limite à d'autres souches bactériennes appartenant à la même espèce ou à une espèce proche de la souche productrice de bactériocine. Les bactériocines peuvent être divisées en deux catégories en fonction de leur poids moléculaire.

En général, les bactériocines de haut poids moléculaire, appelées aussi bactériocine de type queue de phage, sont produites par des bactéries du genre Pseudomonas et par des Entérobactériacées.

On connaît des bactéries du genre Erwinia dont les espèces européennes peuvent être divisées en trois groupes: E. amylovora, E. carotovora et E. herbicola

E. amylovora est responsable du feu bactérien chez les Pomoïdeae (Cotoneaster, Crataegus, Malus, Pyrus, Sorbus, Stranvaesia, Pyracantha) et chez certaines espèces de la sous-famille des Rosaceae.

E.carotovora est l'agent responsable des pourritures molles que l'on observe sur les organes charnus tels que bulbes, racines tubérisées, rhizomes, tubercules.

La sous-espèce E. carotovora ssp atroseptica est responsable de la maladie de la jambe noire de la pomme de terre; elle affecte aussi la betterave, le céleri, le saintpaulia.

E. herbicola est commune à la surface des organes aériens des végétaux, c'est un pathogène opportuniste sur de nombreuses plantes.

Les moyens actuels de lutte contre ces agents phytopathogènes reposent sur un ensemble de mesures prophylactiques destinées à minimiser les risques d'infection primaires et à empêcher l'extension de la maladie en détruisant les parties atteintes. La taille des organes infectés, la scarification des chancres et leur désinfection doivent tenir compte de la progression systémique de la maladie et devancer le front d'avancement des symptômes.

La lutte chimique contre le feu bactérien exige plusieurs traitements par an. Les produits connus actuellement ne réunissent pas l'efficacité, la compatibilité avec d'autres produits, l'absence de phytotoxicité et l'inocuité pour l'environnement. En effet, les sels de cuivre sont peu efficaces et phytotoxiques. La streptomycine est efficace mais son utilisation n'est autorisée que lors de la première floraison. De plus, son action est non spécifique et peut avoir un impact négatif sur la microflore bénéfique. Par l'emploi de streptomycine depuis de nombreuses années aux Etats-Unis, il est apparu des souches résistantes. La streptomycine étant également utilisée en médecine humaine et vétérinaire, des risques de transfert de la résistance aux pathogènes chez l'homme ou l'animal sont possibles.

L'utilisation de bactériophages ou de bactéries antagonistes pour limiter les dégâts causés par E. amylovora ont aussi été expérimentés mais l'application pratique n'a pas donné de résultats probants.

On a résolu ce problème suivant l'invention par une bactériocine appartenant au groupe des bactériocines de haut poids moléculaire (BHPM), caractérisée en ce qu'elle comprend au moins une première sous-unité majeure dont l'extrémité amino-terminale présente une homologie avec une séquence amino-terminale de protéine de tube du phage 186 (SEQ ID N°2), et une seconde sous-unité majeure dont l'extrémité amino-terminale présente une homologie avec une séquence amino-terminale de protéine de manchon du phage P2.

Les deux sous-unités majeures de la bactériocine suivant l'invention sont, de préférence, caractérisées en ce que la première sous-unité est de 23 Kda et la seconde sous-unité est de 43 Kda.

Les séquences correspondantes sont indiquées ci-dessous et les informations les concernant sont annexées:

Séquence de l'extrémité amino-terminale de la première sous-unité majeure de 23 Kda de la bactériocine suivant l'invention (SEQ ID NO: 1)

SEQUENCE: SEQ ID NO: 1:

Ala Leu Pro Lys Lys Leu Lys Tyr Leu Asn Leu Phe Asn Asp Gly Phe
1                    5                    10                    15

Asn Tyr Met Gly Val Val
20

Séquence de l'extrémité amino-terminale de la protéine de tube du phage 186 (SEQ ID NO: 2) (Bertani et Six, 1988; Temple et col., 1991)

SEQUENCE: SEQ ID NO: 2:

Ala Leu Pro Arg Lys Leu Lys Tyr Leu Asn Met Phe Asn Asp Gly Leu
1           5                   10                  15

Ser Tyr Met Gly Val Val
20

Séquence de l'extrémité amino-terminale de la seconde sous-unité majeure de 43 Kda de la bactériocine suivant l'invention (SEQ ID NO: 3)

SEQUENCE: SEQ ID NO: 3:

Asp Tyr His His Gly Val Arg Val Leu
1           5

Séquence de l'extrémité amino-terminale de la protéine du manchon du phage P2 (SEQ ID NO: 4) (Bertani et Six, 1988; Temple et col., 1991)

SEQUENCE: SEQ ID NO: 4:

Asp Tyr His His Gly Val Gln Val Leu
1           5

La séquence amino-terminale de la sous-unité majeure de 23 Kda de la bactériocine suivant l'invention (SEQ ID NO: 1) et la séquence amino-terminale de la protéine du tube interne du phage 186 (SEQ ID NO: 2) présentent une homologie avec un degré d'identité des séquences de 82%.

La séquence amino-terminale de la seconde sous-unité majeure de 43 Kda de la bactériocine suivant l'invention (SEQ ID NO: 3) et l'a séquence amino-terminale de la protéine du manchon de la queue du phage P2 (SEQ ID NO: 4) présentent une homologie avec un degré d'identité des séquences de 88%.

Par homologie, on entend une similitude des séquences mentionnées ci-dessus, ce fait s'ajoutant à une morphologie similaire des protéines et à des réactions immunologiques croisées. On peut en fait suggérer l'existence d'un ancêtre commun à ladite BHPM, au phage 186 et au phage P2 (Bertani et Six, 1988; Temple et col., 1991).

La bactériocine suivant l'invention a une efficacité similaire à celle de la streptomycine sans en présenter les inconvénients.

L'invention concerne également une souche productrice d'une telle bactériocine dont la sélection est décrite ci-dessous:.

Sélection de souches bactériennes produisant des bactériocines de haut poids moléculaire (BHPM)

Il s'avère que les bactériocines sont souvent associées aux entérobactéries. C'est pourquoi des entérobactéries sont isolées à partir de milieux naturels. Elles sont d'abord mises en culture sur un milieu VRBD (Violet Red Bile Dextrose) (Merck) (7 g de peptone de viande; 3 g d'extrait de levure; 5 g de NaCl; 10 g de glucose; 1,5 g de mélange de sels biliaires; 0,03 g de rouge neutre; 0,002 g de cristal violet; 13 g d'agar) pendant 24 heures à 37°C puis les entérobactéries que l'on reconnaît au halo caractéristique qui les entourent sont repiquées sur milieu PCA (Plate Counting Agar) (5 g d'hydrolysat pancréatique de caséine; 2,5 g d'extrait de levure; 1 g de glucose; 15 g d'agar; pH 7). On sélectionne ensuite les souches lysogènes qui sont susceptibles de produire des bactériocines: une colonie isolée de la souche à tester est mise en culture dans 10 ml de milieu 868, en milieu de phase exponentielle, 2 ml de culture sont prélevés et mis dans deux tubes stériles à raison d'un ml par tube. 1 mg de mitomycine C est ajoutée dans chaque tube et ceux-ci sont incubés pendant une nuit à 30°C sans agitation. La phosphatase alcaline est un indicateur de lyse cellulaire et elle est facilement décelable grâce à une réaction colorimétrique avec son substrat, le BCIP. Une solution de BCIP (5-Bromo-4-Chloro-3-Indolyl-Phosphate) est donc ajoutée aux tubes avec des concentrations finales de (0; 35,2; 87; 116 μg/ml).Le BCIP est préparé en mélangeant 35μl de solution stock de BCIP (50 mg/ml) avec 10 ml de tampon (10 mM Tris pH9,5; 100mM NaCl; 15 mM MgCl$_2$). Après 7 heures d'incubation à 30°C sans agitation, la coloration des tubes est observée. Les souches sélectionnées sont observées au MET avec une coloration négative à l'acide phosphotungstique, suivant la méthode de Brenner et Horne (1959). Les souches sont aussi soumises à des tests d'identification préliminaire connus en microbiologie: détermination de la taille, de la forme, de la mobilité et de la présence éventuelle de spores par observation au microscope optique, coloration Gram, réaction d'oxydase, de catalase, acidification du glucose en aérobie et en anaérobie. La composition en acides gras de la membrane cellulaire est déterminée par chromatographie en phase gazeuse avec le système d'identification MIDI commercialement disponible (Microbial Identification System, Inc., Delaware USA). Puis la capacité à oxyder différentes sources de carbone est testée avec le test BIOLOG selon les instructions du fabricant (BIOLOG,Inc., California, USA). Enfin des tests de fermentation et/ou d'assimilation sont réalisées avec des galeries API selon les instructions du fabricant API SYSTEM S.A., La Balme les Grottes, Montalieu Vercieu, France.

Une souche particulièrement appropriée d'entérobactérie Serratia plymuthica a été isolée et sélectionnée de cette manière puis déposée selon le traité de Budapest au BCCM (Belgian Coordinated Collections of Microorganisms) avec le numéro d'accès LMG P-18951, le 2 juin 1999. Cette souche est capable de produire une BHPM qui sera appelée, dans la suite de cette description, la serracine P.

Observation au microscope électronique à transmission (MET) de la serracine P.

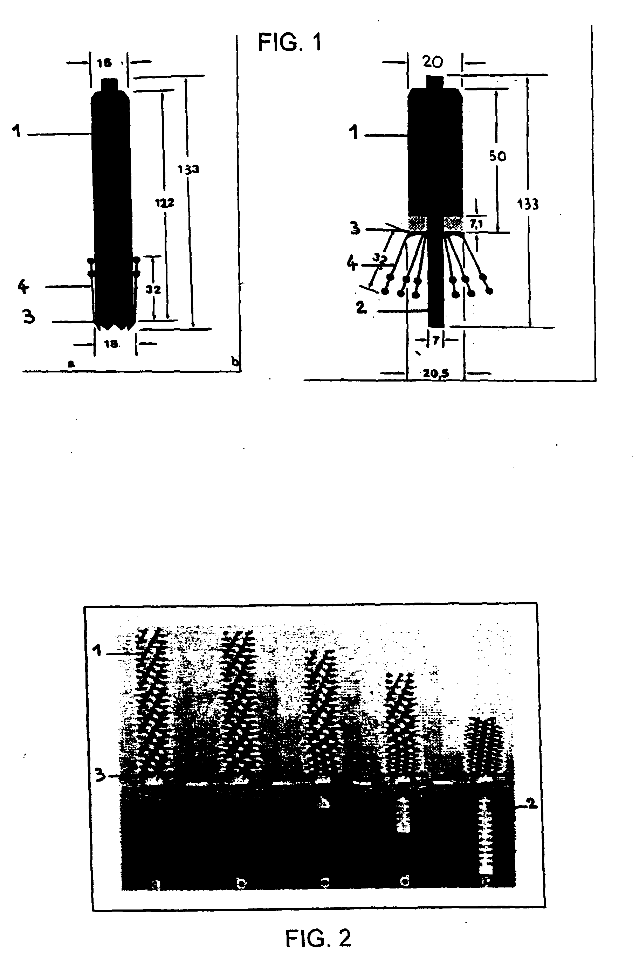

Ainsi qu'il ressort de la figure 1, la serracine P observée au microscope électronique à transmission (MET) suivant la méthode de la coloration négative à l'acide phosphotungstique de Brenner et Home (1959) est caractérisée en ce qu'elle présente la morphologie d'une queue de phage constituée d'un manchon contractile (1) à l'intérieur duquel se trouve le tube interne (2) terminé par une plaque basale (3) comportant des fibres (4). Cette structure, dont les dimensions sont résumées dans le Tableau 3, permet à la serracine P de se fixer et d'agir sur une bactérie-cible de la manière illustrée sur la figure 2: les fibres (4) reconnaissent les lipopolysaccharides à la surface de la bactérie-cible, s'y fixent, et la plaque basale (3) s'adsorbe à la surface de la cellule-cible (a, b) et entraîne la contraction du manchon (1, c), ce qui fait pénétrer le tube interne (2, c, d, e) dans la membrane cytoplasmique de la cellule-cible.

L'invention a aussi pour objet un procédé de production d'une bactériocine suivant l'invention à partir d'une souche de Serratia plymuthica. Un tel procédé peut comprendre avantageusement les étapes suivantes:

a) dans un milieu comprenant une culture de Serratia plymuthica, production de la bactériocine par Serratia plymuthica et
b) induction d'un taux de bactériocine accru dans ce milieu de culture et
c) extraction purification de la bactériocine obtenue, à partir du milieu de culture de Serratia plymuthica.

Induction de la production de bactériocine par Serratia plymuthica.

La production de bactériocine nécessite généralement une induction. Une culture en fermenteur de 20 L d'une souche de Serratia plymuthica. telle qu'isolée ci-dessus, est inoculée à 4% à partir d'une préculture dans du milieu 863 (pour 1 L: 10 g de bactopeptone, 10 g d'extrait de levure, 10 g de glucose, pH 7), après incubation pendant trois heures à 30°C à 300 tpm, avec un volume d'air de 0,5 vvm (volume d'air par volume de culture et par minute). La culture en milieu de phase exponentielle de croissance est induite par tout moyen connu de l'homme du métier, par exemple: addition de mitomycine (0,5 mg/l), ou addition de peroxyde d'hydrogène à 3%, ou addition d'acide nalidixique (2mg/ml) ou par un choc thermique en exposant la culture à une température de l'ordre de 45°C pendant quelques secondes. L'induction active le système SOS de la bactérie (Kenyon C.J., TIBS,8 n°3,1983: 84-97) et provoque une production élevée de bactériocine (environ 22.10$^3$ UA) (UA= unités arbitraires). Après induction, la culture est incubée à 30°C pendant une nuit puis elle est centrifugée à 7000g pendant 20 min à 4°C. Le surnageant est ensuite filtré avec des acrodisc de 0,45μm (Gelman) et le filtrat est stocké à 4°C.

Détermination de l'activité bactéricide du surnageant de Serratia plymuthica.

La méthode utilisée pour déterminer l'activité bactéricide du surnageant de Serratia plymuthica récolté comme décrit ci-dessus, est la méthode de la dilution critique:

a) 200μl d'une suspension de bactéries sensibles d'environ 1.10$^9$ ufc/ml (ufc = unités formatrices de colonies), sont mis dans des tubes contenant 15 ml de milieu 868 semi-solide (7 à 8 g d'agar/l de milieu) maintenu en surfusion (30 à 40°C) puis coulé dans une boîte de Petri.
b) Après 30 minutes, des gouttes de 5 à 10 μl des dilutions successives de la solution de bactériocine sont déposées et laissées sécher (environ 30 minutes).

c) Les boîtes de Petri sont incubées pendant 6 à 10 heures à 27°C puis les zones de lyse provoquées par la bactériocine sont observées. La dilution critique est définie comme la plus grande dilution produisant une zone de lyse. Elle est déterminée sur base de cinq répétitions réalisées pour chaque dilution de la solution de bactériocine. Le titre de la solution de bactériocine, exprimé en UA est égal au facteur de dilution de la dilution critique (Parente E., Brienza C., et Moles M., J. Microbiol. Meth., 22: 95-108, 1995).

Le spectre d'action du surnageant de Serratia plymuthica est testé avec la méthode décrite ci-dessus et les résultats sont montrés dans le Tableau 1. L'action bactéricide du surnageant de Serratia plymuthica vis-à-vis de différentes souches d'Erwinia amylovora isolées en verger en Belgique et dans les pays limitrophes, a été montré en suivant le même protocole et les résultats obtenus sont montrés dans le Tableau 2.

Tableau 1:

| Spectre d'action du surnageant induit de Serratia plymuthica | |
| --- | --- |
| Bactéries | souches sensibles/souches testées (nombre) |
| *Pseudomonas fluorescens*[a] | 1/20 |
| *Pseudomonas putida*[a] | 0/5 |
| *Pseudomonas aeruginosa*[a] | 0/5 |
| *Proteus mirabilis*[b] | 0/1 |
| *Proteus vulgaris*[b] | 0/1 |
| *Klebsiella pneumoniae*[b] | 1/3 |
| *Salmonella sp. HR 22*[b] | 0/1 |
| *Escherichia coli*[b] | 0/2 |
| *Serratia liquefaciens*[b] | 1/1 |
| *Serratia marcescens*[b] | 2/6 |
| *Serratia plymithicum*[d] | 0/1 |
| *Erwinia amylovora*[c] | 1/1 |
| *Erwinia herbicola*[c] | 1/1 |
| *Vibrio sp.*[c] | 0/1 |
| *Thiospherae sp.*[c] | 0/1 |
| *Arthrobacter sp.*[c] | 0/1 |
| *Bacillus megaterium*[c] | 0/1 |
| *Bacillus subtillis BN01*[c] | 0/1 |
| *Micrococcus sp.*[c] | 0/1 |
| *Sarcina sp.*[c] | 0/1 |
| *Staphylococcus aureus* (ATCC 12598) | 0/1 |

[a] isolées de la rhizosphère du peuplier
[b] Unité de Bactériologie et de Pathologie des Maladies Bactériennes
[c] Université de Liège, Belgique
[d] souche utilisée pour la production de BHPM

Tableau 2:

| Activité antibactérienne des surnageants induits et non-induits de Serratia plymuthica vis-à-vis de différentes souches d'Erwinia amylovora. | | | | | |
| --- | --- | --- | --- | --- | --- |
| N° souches | synonymes | plantes hôte | origine | activité antibact. surnageant induit (AU) | surnageant non induit (AU) |
| 2066 LMG | NCPPB 1734 | Pyrus communis | M. Abo-el-Dahab (1968), Egypte | 2187 | 27 |

Tableau 2: (suite)

| Activité antibactérienne des surnageants induits et non-induits de <u>Serratia plymuthica</u> vis-à-vis de différentes souches d'Erwinia amylovora. | | | | | |
|---|---|---|---|---|---|
| N° souches | synonymes | plantes hôte | origine | activité antibact. surnageant induit (AU) | surnageant non induit (AU) |
| 2078 LMG | NCPPB 2135 | Pyrus communis | A. Burckovitcz (1952), Pologne | 723 | 9 |
| 2022 LMG | NCPPB 311 | Pyrus communis | H. Mc. Latry (1952), R.U. | 2187 | 27 |
| 2024 TLMG | NCPPB 683 = ATCC 155580 | Pyrus communis | R. Lelliot (1959), R.U. | 2187 | 27 |
| PD 437 | | Pyrus communis "Clapps Favorite" | J. D. Janse, Pays-Bas | 2187 | 27 |
| 1877 LMG | | Cydonia oblonga | J. Bech-Andersen (1972), Canada | 2187 | 27 |
| 1983 LMG | | Malus sylvestris | G. Bonn (1974), Canada | 2187 | 27 |
| 1969 LMG | | Malus sylvestris cv Golden Delicious | J. Paulin INRA (1980), France | 2187 | 9 |
| 1879 LMG | | Crataegus sp. (bligherd shoots) | J. Hockenhull (1974), Danemark | 2187 | 27 |
| 1965 LMG | | Pyrus communis | M. Ridé (1972), France | 2187 | 27 |
| 2074 LMG | NCPPB 2080 | Pyrus communis (shoot) | D. Dye (1955), Nelle Zélande | 2187 | 27 |
| 1988 LMG | | Pyracantha sp. | IPO (1973), Pays-Bas | 2187 | 27 |
| 1990 LMG | IPO 210 | Sorbus aria | Pays-Bas | 2187 | 27 |
| 2014 LMG | PD 81/683 | Stranvaesia davidiana | Pays-Bas | 2187 | 27 |
| 2015 LMG | PD 81/726 | Cotoneaster dammeri | Pays-Bas | 243 | 3 |
| 2068 LMG | NCPPB 1819 | Crataegus sp. | E.U. | 723 | 9 |
| 2090 LMG | NCPPB 690 | Sorbus aria | R.U. | 2187 | 27 |
| 1884 LMG | VT5 | Cotoneaster salicifolius var. floccosu | Université Gand Belgique (1980) | 2187 | 27 |
| 2057 LMG | NCPPB 1657= Hayward B 229 | Stranvaesia davidiana var. undalata | R.U. | 2187 | 27 |
| 1225 CFBC | NCPPB 2082 | Malus sylvestris | W.J.Kemps (1963) Nelle Zélande | 2187 | 27 |

Tableau 2:   (suite)

| N° souches | synonymes | plantes hôte | origine | activité antibact. surnageant induit (AU) | surnageant non induit (AU) |
|---|---|---|---|---|---|
| | | Activité antibactérienne des surnageants induits et non-induits de Serratia plymuthica vis-à-vis de différentes souches d'Erwinia amylovora. | | | |
| 1232 CFBC | NCPPB 683 = ATCC 155580 | Pyrus communis | R. A. Lelliot (1959), R.U. | 2187 | 27 |
| 1397 CFBC | | Crataegus sp. | J. Bech- Andersen Danemark | 2187 | 27 |
| 1540 CFBC | | Pyrus communis | M. N. Scroth (1973), E.U. | 2187 | 27 |
| 2581 CFBC | | Pyrus sp. | J. P. Paulin (1986), Suède | 2187 | 27 |

Extraction et Purification de la bactériocine (BHPM) produite par Serratia plymuthica

Le surnageant de Serratia Plymuthica présentant une activité bactéricide dont le spectre d'action correspond à celui d'une BHPM, est alors soumis à une nouvelle méthode permettant de purifier la BHPM présente dans ledit surnageant. La première étape de purification est une chromatographie échangeuse d'ions (Mono Q) suivie d'une filtration sur gel (Séphacryl S1000). Cette méthode de purification est simple à mettre en oeuvre, rapide et facilement reproductible.

Le surnageant d'une culture induite de Serratia plymuthica obtenu après centrifugation est soumis à une ultrafiltration sur minicassette Pellicon (Millipore) avec un seuil de coupure de 10000 Da contre du tampon Tris-HCl (20 mM, pH7,7). Le retentat obtenu est centrifugé à 7000 g pendant 20 minutes à 4°C puis filtré sur acrodisc Gelman de 0,45 µm et stocké à 4°C.

Le retentat d'ultrafiltration est alors déposé sur une colonne MonoQ HR 5/5 (Pharmacia) avec un gradient de NaCl réalisé avec un système FPLC. Les fractions de 2,5 ml récoltées (débit de 0,5 ml/min) sont analysées à 280 nm et leur activité bactéricide est déterminée suivant la méthode décrite précédemment. Les fractions contenant la bactériocine sont regroupées puis concentrées par ultracentrifugation, comme décrit précédemment. Le culot est remis en suspension dans 20 mM de tampon Tris-HCl, 0,15 M NaCl, pH 7,7 et filtré sur acrodisc Gelman 0,45 µm avant d'être injecté dans une colonne de chromatographie à tamis moléculaire (Séphacryl S1000 superfine , Pharmacia). La colonne est reliée à un système FPLC. L'élution des molécules est réalisée avec un tampon Tris-HCl (débit 0,7 ml/min). Les fractions récoltées sont analysées pour leur activité bactéricide comme décrit précédemment.

Le dosage des protéines est effectué suivant la méthode colorimétrique de Bradford (1978). La pureté de la bactériocine obtenue est analysée par électrofocalisation suivant la méthode de Wiktorsson et col., (1992).

Caractérisation physique et enzymatique de la serracine P:

Les fractions de bactériocine purifiée obtenue par chromatographie à tamis moléculaire sont traitées avec les enzymes suivantes: papaïne (Merck): 0,05 mg/ml, 30 min, 25°C; lipase (Fluka): 0,05 mg/ml, 120 min, 37°C; pronase E (Sigma): 0,05 mg/ml, 10 min, 37°C; trypsine (Sigma): 2,5 mg/ml, 60 min, 25°C; ribonucléase (Sigma): 5 mg/ml, 60 min, 37°C. Par ailleurs les échantillons de bactériocine ont été incubés pendant 10 min à 40, 50, 60, et 70°C. Après chaque traitement, l'activité bactéricide est mesurée suivant la méthode décrite précédemment.

La détermination du poids moléculaire de la bactériocine suivant l'invention est réalisée suivant la méthode d'électrophorèse en conditions dénaturantes (SDS-PAGE) bien connue de l'homme du métier (Laemmli, Nature, 277: 280, 1970)

Les propriétés physico-chimiques et biochimiques de la bactériocine suivant l'invention sont présentées dans le Tableau 3.

L'électrophorèse en conditions dénaturantes (SDS-PAGE) de la serracine P réalisée ci-dessus a montré l'existence de deux sous-unités majeures de 23 et 43 kDa. Ces deux sous-unités représentent ensemble 75,8% de la structure globale de la bactériocine, il est important d'en déterminer la composition en acides aminés. En effet, d'autres études concernant des BHPM ont montré la présence de deux sous-unités majeures correspondant à la protéine du manchon et à la protéine du tube interne de la structure de queue de phage.

Tableau 3:

| Caractéristiques morphologiques, physico-chimiques et biochimiques de la serracine P. | | |
|---|---|---|
| Etude | MET | -Queue de phage avec queue contractile |
| morphologique | | -Forme relâchée: longueur 133 nm, largeur 16 nm.<br>-Forme contractée: longueur du manchon 50 nm, largeur du manchon 16 nm.<br>- Présence de fibres |
| Caractérisation<br><br>physico-chimique | | -Sensible à une incubation pendant 10 min à une température supérieure à 50°C et à un traitement trypsique.<br>- Résistante à la pronase E, lipase papaïne et à la ribonucléase. |
| | | - Masse moléculaire de $3,2 \times 10^7$ Da |
| Etude biochimique | SDS-PAGE | 2 bandes majeures de 23 et 43 kDa (34,9 et 40,9 %).<br>2 bandes d'intensité moyenne de 32 et 71 kDa (9,8 et 7,3 %)<br>"5 bandes" d'intensité faibles de 30, 34, 38, 42, et 110 kDa (7,3%) |
| | Composition en acides aminées | -23 kDa est riche en glycine, leucine, acide aspartique (plus asparagine), valine et sérine<br>-43 kDa est riche en acide aspartique (plus asparagine), alanine, et leucine |
| | Analyse de la séquence N-terminale | *La séquence N-terminale de 23 kDa est identique à:<br>- protéine du tube interne du phage 186 à 82%.<br>*La séquence N-terminale de 43 kDa est identique à: protéine majeure du manchon de la queue du phage P2 à 88% |

Les différentes sous-unités séparées par électrophorèse suivant la méthode décrite ci-dessus (SDS-PAGE), sont donc transférées sur un filtre de nitrocellulose (Novablot System, Pharmacia) puis elles sont soumises à une hydrolyse acide en phase gazeuse avec de l'HCl 6 N pendant 1 heure à 165°C. Une analyse des acides aminés a ensuite été réalisée avec un derivatizer 420A avec analyse PTC en ligne sur un système de 130 séparations (Perkin-Elmer Applied Biosystems Division). Les résultats sont montrés dans le Tableau 4.

Tableau 4:

| Composition en acides aminés des deux sous-unités majeures de la serracine P | | |
|---|---|---|
| Noms des résidus acides aminés | Nombre de résidus dans les sous-unités majeures de la serracine P | |
| | 23 kDa | 43 kDa |
| Asx | 25 | 50 |
| Ser | 15 | 3 |
| His | 4 | 14 |
| Thr | 11 | 19 |
| Pro | 4 | 11 |
| Ile | 6 | 20 |
| Phe | 6 | 12 |
| Glx | 11 | 12 |

Tableau 4:   (suite)

| Composition en acides aminés des deux sous-unités majeures de la serracine P | | |
|---|---|---|
| Noms des résidus acides aminés | Nombre de résidus dans les sous-unités majeures de la serracine P | |
| | 23 kDa | 43 kDa |
| Gly | 24 | 21 |
| Arg | 14 | 59 |
| Ala | 2 | 36 |
| Tyr | 4 | 13 |
| Val | 11 | 23 |
| Met | 2 | 1 |
| Leu | 15 | 31 |
| Lys | 17 | 64 |

Séquençage des extrémités amino-terminales des deux sous-unités majeures de la serracine P.

Les deux sous-unités séparées précédemment par électrophorèse (SDS-PAGE) et transférées sur filtre (Novablot System, Pharmacia) sont ensuite analysées selon la dégradation d'Edman, à l'aide d'un séquenceur automatique en phase liquide pulsée (Applied Biosystem 476A, Perkin Elmer). Les séquences amino-terminales de ces deux sous-unités (SEQ ID NO: 1 et SEQ NO: 3) qui ont été établies suivant cette méthode sont annexées.

La bactériocine suivant l'invention est produite par une souche bactérienne de Serratia plymuthica déposée le 2 juin 1999 au BCCM (Belgian Coordinated Collections of Microorganisms) avec le numéro d'accès LMG P-18951, présente une activité bactéricide vis-à-vis des bactéries phytopathogènes du genre Erwinia. C'est pourquoi, suivant une forme de réalisation de l'invention, il est prévu une composition phytosanitaire comprenant une bactériocine suivant l'invention et au moins un support acceptable du point de vue phytosanitaire.

Formulation de la serracine P produite par Serratia plymuthica:

La formulation phytopharmaceutique de la serracine P permet son utilisation efficace dans un traitement antiparasitaire tout en restant stable, facile à appliquer et en réduisant les risques de contamination de l'environnement et d'intoxication des utilisateurs. On peut utiliser dans ce but tout support phytosanitaire approprié.

A titre d'exemple, on peut utiliser une solution de bactériocine pour la prophylaxie ou le traitement d'une infection bactérienne chez des espèces végétales, en diluant la substance active dans de l'eau peptonée, et des adjuvants tels que des photoprotecteurs peuvent être incorporés de façon à ce que la matière active soit protégée des radiations UV. Ces adjuvants peuvent être, par exemple: du rouge Congo, de l'acide folique, de l'acide para aminobenzoïque (PABA) ainsi que des chromophores organiques comme: l'acriflavine, le vert de méthyl et la rhodamine B.

De même, pour protéger la serracine P contre les changements d'humidité relative qui affectent son activité, des composés humectants ou formateurs de films peuvent faire partie de la formulation phytopharmaceutique.

D'autres particularités de l'invention sont indiquées dans les revendications annexées.

Les exemples suivants d'utilisation de la serracine P produite par Serratia plymuthica sont donnés à titre illustratif et non pour limiter le cadre de l'invention de quelque manière que ce soit. Les expériences décrites ont été réalisées dans le cadre d'un programme de recherche subventionné par le Ministère des Classes Moyennes et de l'Agriculture.

Exemple 1: Action de la serracine P contre le feu bactérien lors de la floraison principale des poiriers de la variété Conférence

Des fleurs de poiriers sont cueillies puis incisées en oblique et placées dans un erlenmeyer rempli d'eau stérile. Elles sont ensuite aspergées avec une culture bactérienne d'Erwinia amylovora de 24 h et de $10^8$ UFC/ml. Une demi-heure plus tard, les fleurs sont aspergées soit avec une solution de serracine P 2100 UA (100ml/l), soit avec une solution de streptomycine (0,6 g/l), soit avec une formulation de serracine P comprenant 2100UA de serracine P et un fongicide, le captan (1,5 g/ml). On les laisse sécher puis on dépose les erlenmeyer à 24°C en atmosphère humide et les fleurs sont soumises à un cycle jour/nuit de 12 h. Un témoin non traité a été aspergé d'eau stérile et soumis aux même conditions expérimentales.

L'essai est tripliqué et réalisé avec quatre bouquets floraux comportant chacun quatre fleurs par bouquet.

La valeur du taux d'infection pondéré (TH3) est déterminée de la façon suivante:

$$TH3 = \frac{\text{nombre total de points par bouquet}}{\text{nombre maximum de points par bouquet}} \times 100$$

Les points représentent le degré de gravité du feu bactérien:

1 point = coloration noire sur une partie de la fleur
2 points = coloration noire sur toute la fleur
3 points = chancre

Les valeurs moyennes du taux d'infection pondéré sont montrées dans le tableau 5 ci-dessous:

Tableau 5:

| Valeur moyenne du taux d'infection pondéré (TH3) pour chaque objet. | | |
|---|---|---|
| | Concentration | Moyenne du TH3 |
| Contrôle | | 23,24 |
| Streptomycine | 0,6 g/l | 12,73 |
| Serracine P | 100 ml/l | 5,70 |
| Serracine P+Captan | 100 ml/l + 1,5 g/l | 4,31 |

D'après ces résultats, l'action de la bactériocine suivant l'invention est plus efficace que celle de la streptomycine et son effet est amplifié par l'addition de captan à la solution de serracine P.

Exemple 2: Essai en verger de l'action de la serracine P

Le surnageant d'une culturesuivant l'invention de <u>Serratia plymuthica</u> est récupéré suivant la méthode décrite précédemment.

Les essais sont réalisés dans des vergers de poiriers de la variété Durondeau connue pour sa sensibilité au feu bactérien. Les conditions climatologiques ayant une influence sur ces essais, un suivi journalier est effectué en se référant au système BRS (Billing's Revised System) bien connu de l'homme du métier.

Cet essai est réalisé dans deux parcelles d'un même verger, chaque parcelle comprenant 10 arbres.

La serracine P et la streptomycine sont testées à des dilutions finales respectives de $2,2 \times 10^9$ UA/ha et 100 g/ha. Des dilutions sont réalisées dans l'eau de façon à traiter les arbres avec la même quantité de solution (300 l/ha) pour les deux substances testées. Les paramètres des essais sont repris dans le tableau 6 ci-dessous:

Tableau 6:

| Paramètres de l'essai en verger sur poiriers de la variété Durondeau | | | | | | |
|---|---|---|---|---|---|---|
| Jours | 1 | 15 | 32 | 45 | 60 | 80 |
| Phénologie | floraison secondaire | | | | | |
| Traitements | x | x | x | x | x | x |
| Serracine P | 7290 UA/ml = $2,2 \times 10^9$ UA/ha | | | | | |
| streptomycine | 100g /ha | | | | | |

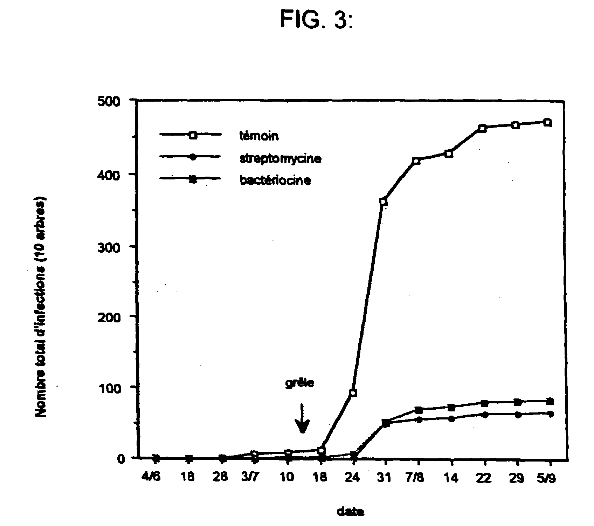

L'évolution du nombre d'infections provoquées par le feu bactérien dans les deux parcelles, est présenté sur la figure 3. On constate une augmentation brutale du nombre d'infections après la période de grêle. Cet essai montre l'action antibactérienne de la bactériocine suivant l'invention, contre Erwinia amylovora, in situ. De plus, son efficacité est comparable à celle de la streptomycine.

LISTE DE SEQUENCES

(1) INFORMATIONS GENERALES:

(i) DEPOSANT:
  (A) NOM: AGROSTAR
  (B) RUE: Avenue Vésale, 30
  (C) VILLE: WAVRE
  (E) PAYS: BELGIQUE
  (F) CODE POSTAL: 1300
  (G) TELEPHONE: 32 10 22 83 34
  (H) TELECOPIE: 32 10 22 83 38

(ii) TITRE DE L' INVENTION: BACTERIOCINE,SA PREPARATION ET SON UTILISATION

(iii) NOMBRE DE SEQUENCES: 4

(iv) FORME DECHIFFRABLE PAR ORDINATEUR:
  (A) TYPE DE SUPPORT: Floppy disk
  (B) ORDINATEUR: IBM PC compatible
  (C) SYSTEME D' EXPLOITATION: PC-DOS/MS-DOS
  (D) LOGICIEL: PatentIn Release #1.0, Version #1.30 (OEB)

(2) INFORMATIONS POUR LA SEQ ID NO: 1:

(i) CARACTERISTIQUES DE LA SEQUENCE:
  (A) LONGUEUR: 22 acides aminés
  (B) TYPE: acide aminé,
  (C) NOMBRE DE BRINS:
  (D) CONFIGURATION: linéaire

(ii) TYPE DE MOLECULE: protéine

(v) TYPE DE FRAGMENT: N-terminal

(vi) ORIGINE:
  (B) SOUCHE: Serratia plymuthica

(xi) DESCRIPTION DE LA SEQUENCE: SEQ ID NO: 1:

Ala Leu Pro Lys Lys Leu Lys Tyr Leu Asn Leu Phe Asn Asp Gly Phe
1               5                   10                  15

Asn Tyr Met Gly Val Val
            20

(2) INFORMATIONS POUR LA SEQ ID NO: 2:

(i) CARACTERISTIQUES DE LA SEQUENCE:
  (A) LONGUEUR: 22 acides aminés
  (B) TYPE: acide aminé
  (C) NOMBRE DE BRINS:
  (D) CONFIGURATION: linéaire

(ii) TYPE DE MOLECULE: protéine

(v) TYPE DE FRAGMENT: N-terminal

(vi) ORIGINE:
    (A) ORGANISME: Bacteriophage 186

(xi) DESCRIPTION DE LA SEQUENCE: SEQ ID NO: 2:

Ala Leu Pro Arg Lys Leu Lys Tyr Leu Asn Met Phe Asn Asp Gly Leu
1            5          10          15

Ser Tyr Met Gly Val Val
         20

(2) INFORMATIONS POUR LA SEQ ID NO: 3:

    (i) CARACTERISTIQUES DE LA SEQUENCE:
        (A) LONGUEUR: 9 acides aminés
        (B) TYPE: acide aminé,
        (C) NOMBRE DE BRINS:
        (D) CONFIGURATION: linéaire

    (ii) TYPE DE MOLECULE: protéine

    (v) TYPE DE FRAGMENT: N-terminal

    (vi) ORIGINE:
        (A) ORGANISME: Serratia plymuthica

    (xi) DESCRIPTION DE LA SEQUENCE: SEQ ID NO: 3:

    Asp Tyr His His Gly Val Arg Val Leu
    1            5

(2) INFORMATIONS POUR LA SEQ ID NO: 4:

    (i) CARACTERISTIQUES DE LA SEQUENCE:
        (A) LONGUEUR: 9 acides aminés
        (B) TYPE: acide aminé,
        (C) NOMBRE DE BRINS:
        (D) CONFIGURATION: linéaire

    (ii) TYPE DE MOLECULE: protéine

    (v) TYPE DE FRAGMENT: N-terminal

    (vi) ORIGINE:
        (A) ORGANISME: Bacteriophage P2

    (xi) DESCRIPTION DE LA SEQUENCE: SEQ ID NO: 4:

Asp Tyr His His Gly Val Gln Val Leu
1                5

Annexe aux documents de la demande - listage des séquences déposé ultérieurement

Annexe aux documents de la demande - listage des séquences déposé ultérieurement

[0060]

LISTE DE SEQUENCES

<110> AGROSTAR

<120> Bactériocine, sa préparation et son utilisation

<130> Orep125406

<140> 99870124.7
<141> 1999-06-11

<160> 4

<170> PatentIn Ver. 2.1

<210> 1
<211> 22
<212> PRT
<213> Serratia plymuthica

<400> 1
Ala Leu Pro Lys Lys Leu Lys Tyr Leu Asn Leu Phe Asn Asp Gly Phe
1               5                   10                  15

Asn Tyr Met Gly Val Val
                20


<210> 2
<211> 22
<212> PRT
<213> Serratia plymuthica

<400> 2
Ala Leu Pro Arg Lys Leu Lys Tyr Leu Asn Met Phe Asn Asp Gly Leu
1               5                   10                  15

Ser Tyr Met Gly Val Val
                20


<210> 3
<211> 9
<212> PRT
<213> Serratia plymuthica

```
<400> 3
Asp Tyr His His Gly Val Arg Val Leu
  1                   5




<210> 4
<211> 9
<212> PRT
<213> Serratia plymuthica

<400> 4
Asp Tyr His His Gly Val Gln Val Leu
  1                   5
```

**Revendications**

1.  Bactériocine appartenant au groupe des bactériocines de haut poids moléculaire (BHPM), caractérisée en ce qu'elle comprend au moins une première sous-unité majeure dont l'extrémité amino-terminale présente une homologie avec une séquence amino-terminale de protéine de tube de phage 186 (SEQ ID N°2), et une seconde sous-unité majeure dont l'extrémité amino-terminale présente une homologie avec une séquence amino-terminale de protéine de manchon de phage P2 (SEQ ID N°4).

2.  Bactériocine suivant la revendication 1, caractérisée en ce que ladite première sous-unité majeure est de 23 Kda et ladite seconde sous-unité majeure est de 43 Kda.

3.  Bactériocine suivant la revendication 1, caractérisée en ce qu'elle présente une morphologie, observée en microscopie électronique à transmission (MET), de queue de phage avec un manchon contractile.

4.  Bactériocine suivant la revendication 3, caractérisée en ce qu'elle a les propriétés physico-chimiques suivantes:

    i) sensibilité à un traitement avec une protéase, la trypsine
    ii) résistance à l'action des enzymes suivants: pronase E, lipase, papaïne et ribonucléase
    iii) sensibilité àl'exposition à une température de 50°C pendant au moins huit minutes
    iv) poids moléculaire sensiblement égal à 3,2. $10^7$.

5.  Bactériocine suivant la revendication 1, caractérisée en ce qu'elle présente une activité phytosanitaire.

6.  Bactériocine suivant la revendication 5, caractérisée en ce qu'elle présente une activité bactéricide vis-à-vis des bactéries phytopathogènes du genre Erwinia.

7.  Composition phytosanitaire comprenant au moins une bactériocine suivant l'une quelconque des revendications 1 à 6, et au moins un support acceptable du point de vue phytosanitaire.

8.  Bactériocine suivant l'une quelconque des revendications 1 à 4, caractérisée en ce qu'elle est produite par une souche bactérienne de Serratia plymuthica déposée le 2 juin 1999 au BCCM (Belgian Coordinated Collections of Microorganisms) avec le numéro d'accès LMG P-18951.

9.  Souche vivante d'entérobactérie, Serratia plymuthica LMG P-18951 et des variants et mutants dérivés de ladite souche, capable de produire une bactériocine suivant l'une quelconque des revendications 1 à 4.

10. Fragment d'ADN comprenant une séquence codant pour une bactériocine suivant l'une quelconque des revendications 1 à 4.

11. Procédé pour produire une bactériocine suivant l'une quelconque des revendications 1 à 4, comprenant les étapes suivantes:

   a) dans un milieu comprenant une culture de <u>Serratia plymuthica</u>, production de la bactériocine par <u>Serratia plymuthica</u> et
   b) induction d'un taux de bactériocine accru dans ce milieu de culture et,
   c) extraction de la bactériocine obtenue, à partir du milieu de culture de <u>Serratia plymuthica</u>.

12. Utilisation de la bactériocine suivant l'une quelconque des revendications 1 à 4 et 8, pour la prophylaxie ou le traitement d'une infection bactérienne chez des espèces végétales.

13. Utilisation suivant la revendication 12 pour la prophylaxie ou le traitement du feu bactérien chez les arbres fruitiers, les espèces végétales arbustives et les espèces végétales ornementales.

14. Utilisation suivant la revendication 12 pour la prophylaxie ou le traitement de la maladie de la pourriture molle et de la maladie de la jambe noire sur les organes charnus d'espèces végétales.

FIG. 1

FIG. 2

# FIG. 3:

**Office européen des brevets**

# RAPPORT DE RECHERCHE EUROPEENNE

Numéro de la demande

EP 99 87 0124

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | NAKAYAMA K ET AL.: "The complete nucleotide sequence of phiCTX, a cytotoxin-converting phage of Pseudomonas aeruginosa: implication for phage evolution and horizontal gene transfer via bacteriophages" MOLECULAR MICROBIOLOGY, vol. 31, no. 2, janvier 1999 (1999-01), pages 399-419, XP000852986 Remarque: les extrémités amino-terminales des protéines codées par orf22 et orf23 présentent une homologie avec les séquences de SEQ ID NO:2 et SEQ ID NO:4. * page 399, colonne de droite, alinéa 3 * * page 400, colonne de droite, alinéas 3,4 *, phrases 22,23; tableau 1 * * page 404, colonne de gauche, alinéa 3 * * page 407, colonne de droite * * figure 9 * * page 413, colonne de droite - page 414, colonne de gauche, alinéa 1 *<br>--- | 1-3,10 | C12N15/31 C07K14/24 A01N63/02 |
| X | XUE, QING ET AL: "Tail sheath and tail tube genes of the temperate coliphage 186" VIROLOGY (1995), 212(1), 218-21 , XP002122243 * abrégé * * figure 1 *<br>--- | 1-3,10 | **DOMAINES TECHNIQUES RECHERCHES (Int.Cl.7)** C12N C07K A01N |
| | -/-- | | |

Le présent rapport a été établi pour toutes les revendications

| Lieu de la recherche | Date d'achèvement de la recherche | Examinateur |
|---|---|---|
| LA HAYE | 15 novembre 1999 | van de Kamp, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

**EP 1 059 355 A1**

Office européen
des brevets

**RAPPORT DE RECHERCHE EUROPEENNE**

Numéro de la demande

EP 99 87 0124

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,X | TEMPLE, LOUISE M. ET AL: "Nucleotide sequence of the genes encoding the major tail sheath and tail tube proteins of bacteriophage P2" VIROLOGY (1991), 181(1), 353-8 , XP002122244 * abrégé * * page 353 * * page 355, colonne de droite, alinéas 2,3 * * figure 3 * * page 357, colonne de droite, alinéa 3 * | 1-3,10 | |
| A | WO 84 02448 A (UNIVERSITY PATENTS INC) 5 juillet 1984 (1984-07-05) * le document en entier * | 1-14 | |
| A | EP 0 182 106 A (MICROLIFE TECHNICS) 28 mai 1986 (1986-05-28) * le document en entier * | 1-14 | |
| A | SHINOMIYA T ET AL.: "Bactericidal activity of the tail of Pseudomonas aeruginosa bacteriophage PS17" JOURNAL OF VIROLOGY, vol. 32, 1979, pages 958-967, XP002122279 * abrégé * * tableau 1 * * page 963 – page 965 * * figure 6 * | 1-3 | DOMAINES TECHNIQUES RECHERCHES (Int.Cl.7) |

Le présent rapport a été établi pour toutes les revendications

| Lieu de la recherche | Date d'achèvement de la recherche | Examinateur |
|---|---|---|
| LA HAYE | 15 novembre 1999 | van de Kamp, M |

EPO FORM 1503 03.82 (P04C02)

**EP 1 059 355 A1**

### ANNEXE AU RAPPORT DE RECHERCHE EUROPEENNE
### RELATIF A LA DEMANDE DE BREVET EUROPEEN NO.

EP 99 87 0124

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

15-11-1999

| Document brevet cité au rapport de recherche | | Date de publication | Membre(s) de la famille de brevet(s) | | Date de publication |
|---|---|---|---|---|---|
| WO 8402448 | A | 05-07-1984 | CA | 1207255 A | 08-07-1986 |
| | | | EP | 0128954 A | 27-12-1984 |
| EP 0182106 | A | 28-05-1986 | US | 4678750 A | 07-07-1987 |
| | | | AT | 41729 T | 15-04-1989 |
| | | | AU | 570574 B | 17-03-1988 |
| | | | AU | 4648985 A | 24-04-1986 |
| | | | CA | 1246477 A | 13-12-1988 |
| | | | ES | 547967 A | 01-08-1986 |
| | | | ES | 551118 A | 16-05-1988 |
| | | | NZ | 213871 A | 26-07-1991 |
| | | | US | 4783406 A | 08-11-1988 |

EPO FORM P0460

Pour tout renseignement concernant cette annexe : voir Journal Officiel de l'Office européen des brevets, No.12/82

22